(19) Europäisches Patentamt
European Patent Office
Office européen des brevets

(11) Veröffentlichungsnummer: **0 191 443**
**B1**

(12) **EUROPÄISCHE PATENTSCHRIFT**

(45) Veröffentlichungstag der Patentschrift: **18.04.90**

(21) Anmeldenummer: **86101660.8**

(22) Anmeldetag: **10.02.86**

(51) Int. Cl.[5]: **C 07 D 239/46,**
C 07 D 405/12, A 01 N 43/54

# (54) 4-Aminopyrimidine und diese enthaltende Fungizide.

(30) Priorität: **13.02.85 DE 3504895**

(43) Veröffentlichungstag der Anmeldung:
**20.08.86 Patentblatt 86/34**

(45) Bekanntmachung des Hinweises auf die Patenterteilung:
**18.04.90 Patentblatt 90/16**

(84) Benannte Vertragsstaaten:
**AT BE CH DE FR GB IT LI NL SE**

(56) Entgegenhaltungen:
**EP-A-0 000 681**
**EP-A-0 061 019**

**CHEMICAL ABSTRACTS, Band 96, 1982, Seite 764, Zusammenfassung Nr. 142890w, Columbus, Ohio, US**

**CHEMICAL ABSTRACTS, Band 90, 1979, Seite 652, Zusammenfassung Nr. 121638f, Columbus, Ohio, US**

(73) Patentinhaber: **BASF Aktiengesellschaft**
**Carl-Bosch-Strasse 38**
**D-6700 Ludwigshafen (DE)**

(72) Erfinder: **Eicken, Karl, Dr.**
**Am Hüttenwingert 12**
**D-6706 Wachenheim (DE)**
Erfinder: **Wahl, Peter, Dr.**
**Valentinianstrasse 8**
**D-6802 Ladenburg (DE)**
Erfinder: **Ammermann, Eberhard, Dr.**
**Sachsenstrasse 3**
**D-6700 Ludwigshafen (DE)**
Erfinder: **Pommer, Ernst-Heinrich, Dr.**
**Berliner Platz 7**
**D-6703 Limburgerhof (DE)**

(56) References cited:
**JOURNAL OF THE CHEMICAL SOCIETY, 1950, Abschnitt 90, Seiten 452-459, London, GB; J.D. BROOKS et al.: "Antituberculous compounds. Part V. 2-Sulphanilamido-5-alkyl-1:3:4-oxadiazoles and -thiadiazoles and related isothiosemicarbazones and isothioureas"**

**JOURNAL OF ORGANIC CHEMISTRY, Band 24, 1959, Seiten 14-16, Detroit, US; T. OKUDA et al.: "Synthesis of 2-thio analogs of thiamine"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 191 443 B1

**Beschreibung**

Die vorliegende Erfindung betrifft 4-Aminopyrimidine, Verfahren zu ihrer Herstellung und diese enthaltende Fungizide.

Es sind Pyrimidin-Derivate z.B. das 2-(Methylmercapto)-4-methyl-5-n-octyl-6-hydroxypyrimidin (J. Pharm. Soc. Japan, Vol. 96, S. 384 ff (1976) bekannt. Über eine fungizide Wirkung dieser Verbindungen ist nichts bekannt.

Es wurde nun gefunden, daß 4-Aminopyrimidine der Formel

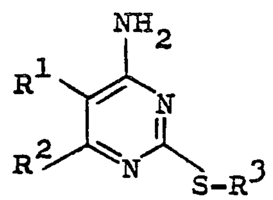

I,

worin

$R^1$ $C_5$—$C_{12}$-Alkyl oder im Phenyl gegebenenfalls durch $C_1$—$C_4$-Alkyl oder Halogen substituiertes Phenyl-$C_2$—$C_6$-alkyl,

$R^2$ Wasserstoff oder $C_1$—$C_4$-Alkyl,

$R^3$ $C_1$—$C_{12}$-Alkyl, $C_3$—$C_8$-Alkenyl, $C_1$—$C_8$-Alkinyl, $C_1$—$C_6$Alkoxi-$C_1$—$C_4$-alkyl, $C_1$—$C_6$-Alkylthio-$C_1$—$C_4$-alkyl, Cyanomethyl, $C_1$—$C_4$-Alkoxicarbonylmethyl, im Phenyl gegebenenfalls durch $C_1$—$C_4$-Alkyl, $C_1$—$C_4$-Alkoxi oder Halogen substituiertes Phenyl-$C_1$—$C_4$-alkyl, Furylmethyl, Tetrahydrofurylmethyl bedeutet, eine gute fungizide Wirkung insbesondere gegen Phycomyceten haben.

Unter den Resten $R^1$ sind $C_5$—$C_{12}$-Alkyl, Phenylalkyl mit 2 bis 6 Kohlenstoffatomen im Alkylteil, z.B. Phenylethyl, Phenylpropyl, Phenylbutyl, Phenylpentyl, Phenylhexyl, welche im Phenyl durch $C_1$—$C_4$-Alkyl, $C_1$—$C_4$-Alkoxi, Fluor, Chlor oder Brom substituiert sein können, zu verstehen. Unter dem Rest $R^2$ ist beispielsweise $C_1$—$C_4$-Alkyl, z.B. Methyl, Ethyl, zu verstehen.

Unter dem Rest $R^3$ ist beispielsweise $C_1$—$C_{12}$-Alkyl, insbesondere $C_1$—$C_6$-Alkyl, z.B. Methyl, Ethyl, $C_1$—$C_6$-Alkoxi-$C_1$—$C_4$-alkyl, insbesondere $C_1$—$C_2$-Alkoxi-$C_1$—$C_4$-Alkyl, $C_1$—$C_6$-Alkylthio-$C_1$—$C_4$-alkyl, Cyanomethyl, $C_1$—$C_4$-Alkoxicarbonylmethyl, $C_3$—$C_8$-Alkenyl, $C_3$—$C_8$-Alkinyl, Phenyl-$C_1$—$C_4$-alkyl, welche im Phenyl durch $C_1$—$C_4$-Alkyl, $C_1$—$C_4$-Alkoxi, Fluor, Chlor oder Brom substituiert sein können (Benzyl), Furylmethyl, Tetrahydrofurylmethyl zu verstehen.

Unter Alkyl oder Alkyl einer Alkoxigruppe ist je nach Zahl der angegebenen Kohlenstoffatomen Methyl, Ethyl, Propyl, Butyl, Pentyl, Hexyl, Heptyl, Octyl, Nonyl, Decyl, Undecyl oder Dodecyl und ihre Isomeren zu verstehen.

Unter Alkenyl bzw. Alkinyl ist je nach Zahl der angegebenen Kohlenstoffatome Allyl, Butenyl, Pentenyl, Heptenyl, Octenyl bzw. Propargyl, Butinyl, Pentinyl, Hexinyl, Heptinyl, Octinyl zu verstehen.

Die Verbindungen mit $R^1$ in der Bedeutung $C_1$—$C_4$-Alkyl, $R^2$ in der Bedeutung Methyl und $R^3$ in der Bedeutung Methyl oder Ethyl sind z.T. bekannt.

Es wurde ferner gefunden, daß man 4-Aminopyrimidine der Formel I erhält, indem man einen substituierten β-Ketoester der Formel II

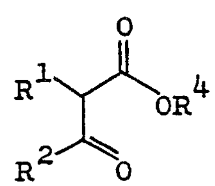

II

in der $R^1$ und $R^2$ die obengenannten Bedeutungen haben und $R^4$ für einen niederen Alkylrest steht, mit Thioharnstoff und Alkalialkoholat in Alkohol zu 2-Thiopyrimidonen der Formel

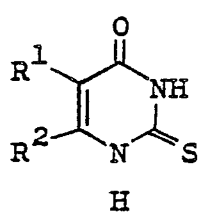

III,

kondensiert, wobei $R^1$ und $R^2$ die in Anspruch I, angegebenen Bedeutungen haben und $R^4$ einen niederen Alkylrest bedeutet, diese mit einer Halogenverbindung $R^3$-Hal, in welcher $R^3$ die in Anspruch 1 angegebene

Bedeutung hat und Hal Chlor, Brom oder Jod bedeutet, zu einem substituierten 2-Thiopyrimidon der Formel

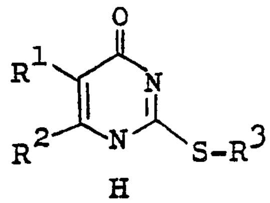

IV,

umsetzt, dieses anschließend mit einem Chlorierungsmittel z.B. Phosphorylchlorid chloriert und das Chlorierungsprodukt mit Ammoniak unter Druck umsetzt. Die β-Ketoester können hergestellt werden wie in Organic Synthesis Coll. Vol. 1, S. 248 beschrieben.

Die Umsetzungen der β-Ketoester mit Thioharnstoff zu den substituierten 2-Thiopyrimidonen der Formel III, deren Umsetzung mit einer Halogenverbindung $R^3$-Hal, in welcher $R^3$ die oben angegebene Bedeutung hat und Hal für Chlor, Brom oder Jod steht, zu einem substituierten 2-Thiopyrimidon der Formel IV, deren Chlorierung mit einem Chlorierungsmittel, vorzugsweise Phosphorylchlorid und die Umsetzung des Chlorierungsproduktes mit Ammoniak unter Druck erfolgen in an sich bekannter Weise (JACS *58*, 1150 (1936); dto *60* 1721 (1938); J. Pharm. Soc. Japan, Vol. *96*, 384 (1976)).

Beispiel

24,1 g 5-n-Octyl-4-oxo-6-methyl-2-methylthiopyrimidin (J. Pharm. Soc. Japan, Vol. *96*, S. 384 (1976)) werden in 150 ml Phosphorylchlorid 30 Minuten am Rückfluß gekocht. Nach dem Verdampfen des Phosphorylchlorids wird der Rückstand mit 150 ml Wasser bei 15—20°C hydrolysiert, in 100 ml Ether aufgenommen und die wäßrige Phase zweimal mit je 30 ml Ether extrahiert. Aus den vereinigten Etherphasen isoliert man nach Waschen mit je 20 ml Wasser, 20 ml Natriumbicarbonat-Lösung, 20 ml Wasser, Trocknen und Verdampfen des Ethers 24,4 g, 5-n-Octyl-4-chlor-6-methyl-2-methylthiopyrimidin als farbloses Öl. (Chlorwert: 12,3%, theor. Wert: 12,36).

10,0 g 5-n-Octyl-4-chlor-6-methyl-2-methylthiopyrimidin, 100 ml Ethanol und 30 ml flüssiger Ammoniak werden in einem Autoklaven (300 ml Volumeninhalt) bei 120°C 38 Stunden gerührt. Nach dem Verdampfen des Lösungsmittels wird der Rückstand mit 100 ml Wasser angeteigt, abgesaugt und getrocknet. Der Rückstand wird in 100 ml Essigester weitgehend gelöst, filtriert und eingeengt. Nach dem Umkristallisieren aus 30 ml Methyl-tert.-butylether isoliert man 5,1 g 5-n-Octyl-4-amino-6-methyl-2-methylthiopyrimidin vom Fp. 119—120°C (Verbindung Nr. 1)

$C_{14}H_{25}N_3S$ (M 267)

| | C | H | N |
|---|---|---|---|
| Theorie | 62,9 | 9,4 | 15,7 |
| Analyse | 63,4 | 9,7 | 16,0 |

Nach dem oben beschriebenen Verfahren können z. B. folgende 4-Amino-pyrimidine hergestellt werden:

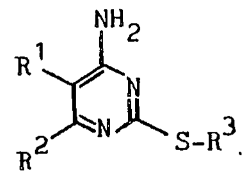

| Nr. | $R^1$ | $R^2$ | $R^3$ | Fp. °C |
|---|---|---|---|---|
| 1 | $n-C_8H_{17}$ | $CH_3$ | $CH_3$ | 119 - 20 |
| 2 | $n-C_8H_{17}$ | $CH_3$ | $C_2H_5$ | 79 - 80 |
| 3 | $n-C_8H_{17}$ | $CH_3$ | $n-C_3H_7$ | 66 - 67 |
| 4 | $n-C_8H_{17}$ | $CH_3$ | $i-C_3H_7$ | 74 - 76 |
| 5 | $n-C_8H_{17}$ | $CH_3$ | $n-C_6H_{13}$ | 60 - 62 |
| 6 | $n-C_8H_{17}$ | $CH_3$ | $i-C_4H_9$ | 69 - 70 |
| 7 | $n-C_8H_{17}$ | $CH_3$ | $CH_2-CH=CH_2$ | 83 - 84 |
| 8 | $n-C_8H_{17}$ | $CH_3$ | $CH_2-C(CH_3)=CH_2$ | 58 - 60 |
| 9 | $n-C_8H_{17}$ | $CH_3$ | $(CH_2)_2-CH=CH_2$ | 44 - 45 |
| 10 | $n-C_8H_{17}$ | $CH_3$ | $CH_2CH=CH-CH_3$ | 65 - 67 |
| 11 | $n-C_8H_{17}$ | $CH_3$ | Propargyl | |
| 12 | $n-C_8H_{17}$ | $CH_3$ | 2-butin-1-yl | |
| 13 | $n-C_8H_{17}$ | $CH_3$ | 3-butin-2-yl | |
| 14 | $n-C_9H_{19}$ | $CH_3$ | $CH_3$ | 115 - 117 |
| 15 | $n-C_5H_{11}$ | $CH_3$ | $CH_3$ | 108 - 109 |
| 16 | $n-C_6H_{13}$ | $CH_3$ | $CH_3$ | 107 - 108 |
| 17 | $CH_3(CH_2)_3-CH(C_2H_5)-CH_2$ | $CH_3$ | $CH_3$ | |
| 18 | $(CH_3)_3CCH_2-CH(CH_3)-(CH_2)_2$ | $CH_3$ | $CH_3$ | 98 - 99 |
| 19 | $n-C_{10}H_{21}$ | $CH_3$ | $CH_3$ | |
| 20 | $n-C_{12}H_{25}$ | $CH_3$ | $CH_3$ | |

| Nr. | $R^1$ | $R^2$ | $R^3$ | Fp. °C |
|---|---|---|---|---|
| 21 | $n-C_8H_{17}$ | $CH_3$ | $(CH_2)_2OCH_3$ | 54 - 61 |
| 22 | $n-C_8H_{17}$ | $CH_3$ | $(CH_2)_2OC_2H_5$ | 38 - 39 |
| 23 | $n-C_8H_{17}$ | $CH_3$ | $CH(CH_3)CH_2OCH_3$ | 81 |
| 24 | $n-C_8H_{17}$ | $CH_3$ | $CH_2OCH_3$ | |
| 25 | $n-C_8H_{17}$ | $CH_3$ | $CH_2SCH_3$ | |
| 26 | $n-C_8H_{17}$ | $CH_3$ | $(CH_2)_2SC_2H_5$ | 74 - 75 |
| 27 | $n-C_8H_{17}$ | $CH_3$ | 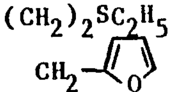 | |
| 28 | $n-C_8H_{17}$ | $CH_3$ | 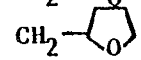 | 81 |
| 29 | $n-C_8H_{17}$ | $CH_3$ | 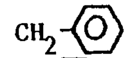 | 83 |
| 30 | $n-C_8H_{17}$ | $CH_3$ | 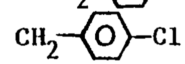 | 79 |
| 31 | $n-C_8H_{17}$ | $CH_3$ | 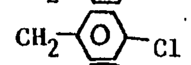 | 70 |
| 32 | $n-C_8H_{17}$ | $CH_3$ | 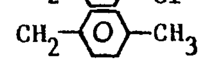 | |
| 33 | $n-C_8H_{17}$ | $CH_3$ | 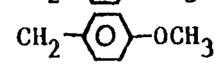 | |
| 34 | 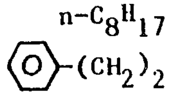 | $CH_3$ | $CH_3$ | |
| 35 | 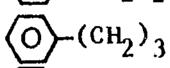 | $CH_3$ | $CH_3$ | 126 |
| 36 | 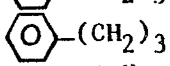 | $CH_3$ | $CH_2-CH=CH_2$ | 86 - 88 |
| 37 | $n-C_7H_{15}$ | $CH_3$ | $CH_3$ | |
| 38 | $n-C_4H_9$ | $n-C_4H_5$ | $CH_3$ | |
| 39 | $n-C_4H_9$ | $n-C_3H_7$ | $CH_3$ | |
| 40 | $n-C_8H_{17}$ | $C_2H_5$ | $CH_3$ | |
| 41 | $n-C_8H_{17}$ | $CH_3$ | $CH_2-CN$ | |
| 42 | $n-C_8H_{17}$ | $CH_3$ | $CH_2-CO_2CH_3$ | |
| 43 | $n-C_8H_{17}$ | $CH_3$ | $CH_2-CO_2C_2H_5$ | |

Die neuen Wirkstoffe zeigen eine starke fungitoxische Wirksamkeit gegen phytopathogene Pilze, insbesondere aus der Klasse der Phycomyceten. Die neuen Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben sowie von Erysiphe graminis an Getreide, Uncinula necator an Reben, Podophaera leucotricha an Äpfeln, Sphaerotheca fuliginea an Rosen, Erysiphe cichoracearum an Gurken.

Die Wirkstoffe besitzen eine hohe Pflanzenverträglichkeit. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen.

Die fungiziden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,1 bis 5 kg Wirkstoff je ha.

Die Wirkstoffe können auch zusammen mit anderen Wirkstoffen, z.B. Herbiziden, Insektiziden, Wachstrumsregulatoren und anderen Fungiziden, oder auch mit Düngemitteln vermischt ausgebracht werden. In vielen Fällen erhält man bei der Mischung mit Fungiziden auch eine Vergrößerung des fungiziden Wirkungsspektrum; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten.

Fungizide, die mit den neuen Verbindungen kombiniert werden können, sind beispielsweise:
Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethyl-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyron-3-carbonsäure-anilid

2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-(Chlorphenoxy)-3,3-dimethyl-1-(1H-1,2,4-triazol-1-yl)-2-butanon
1-(4-(Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
2-Cyano-N-(ethylaminocarbonyl)-2-(methoximino)-acetamid

sowie verschiedene Fungizide, wie
Dodecylguanidinacetat
3-(3-(3,5-Dimethyl-2-oxycyclohexyl)-2-hydroxyethyl)-glutaramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid
2-Cyano-[N-(ethylaminocarbonyl)-2-methoximinol]-actamid
1-[2-(2,4-Dichlorphenyl)-pentyl]-1H-1,2,4-triazol
2,4-difluor-alpha-(1H-1,2,4-triazolyl-1-methyl)-benzhydrylalkohol.

Die neuen Wirkstoffe werden beispielsweise in Form von direkt versprühbaren Lösungen, Suspensionen, Emulsionen, auch in Form von hochprozentigen wäßrigen, öligen oder sonstigen Dispersionen, Pasten, Stäubemitteln, Streumitteln, Granulaten durch Versprühen, Verstäuben, Verstreuen, Verstreichen oder Gießen ausgebracht. Die Anwendungsformen richten sich ganz nach den Verwendungszwecken; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie kerosin oder Dieselöl, ferner Kohlenteeröle, sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem

Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden.

Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Beispiele für solche Pflanzenschutzmittel-Zubereitungen sind:

I. Man vermischt 90 Gewchtsteile der Verbindung des Beispiels 1 mit 100 Gewichtsteilen N-Methylpyrrolidon und erhält eine Lösung, die zur Anwendung in Form kleinster Tropfen geeignet ist.

II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Doedecylbenzolsulfonsäure und 5 Gewichtsteilen des Anlagerungsproduktes von 40 Mol Ethylenoxid an 1 Mol Ricinusöl besteht. Durch Ausgießen und feines Verteilen der LÖsung in Wasser erhält man eine wäßrige Dispersion.

III. 20 Gewichtsteile der nach Beispiel 7 erhältlichen Verbindung werden in einer Mischung gelöst, die aus 30 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und Verrühren der Lösung in Wasser erhält man eine wäßrige Dispersion.

IV. 20 Gewichtsteile der nach Beispiel 9 erhältlichen Verbindung werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und Verrühren der Lösung in Wasser, erhält man eine wäßrige Dispersion.

V. 20 Gewichtsteile der nach Beispiel 10 erhältlichen Verbindung werden mit 3 Gewichtsteilen des Natriumsalzes der Diisobutylnaphthalin-alphasulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der nach Beispiel 14 erhältlichen Verbindung werden mit 95 Gewichtsteilen feinteiligem Kaolin innig vermischt. Man erhält auf diese Weise ein Stäubemittel, das 5 Gew.-% des Wirkstoffs enthält.

VII. 30 Gewichtsteile der nach Beispiel 21 erhältlichen Verbindung werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der nach Beispiel 22 erhältlichen Verbindung werden mit 30 Teilen Natriumsalz eines Phenolsulfonsäure-Harnstoff-Formaldehyd-Kondensates, 2 Teilen Kieselgel und 48 Teilen Wasser innig vermischt. Man erhält eine stabile wäßrige Dispersion.

IX. 20 Teile der nach Beispiel 28 erhältlichen Verbindung werden mit 2 Teilen Calciumsalz der Dodecylbenzolsulfonsäure, 8 Teilen Fettalkoholpolyglykolether, 2 Teilen Natriumsalz eines Phenolsulfonsäureharnstoff-formaldehyd-Kondensates und 68 Teilen eines paraffinischen Mineralöls innig vermischt. Man erhält eine stabile ölige Dispersion.

Der folgende Versuch belegt die biologische Wirkung der neuen Verbindungen. Als Vergleichsmittel wurde die bekannte Verbindung 2-(Methylmercapto)-4-methyl-5-n-octyl-6-hydroxypyrimidin verwendet.

## Anwendungsbeispiel

Wirksamkeit gegen Plasmopara viticola

Blätter von Topfreben der Sorte "Müller-Thurgau" werden mit wäßriger Spritzbrühe, die 80% Wirkstoff und 20% Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann

erfolgt die Beurteilung des Ausmaßes des Pilzausbruches auf den blattunterseiten.

Das Ergebnis zeigt, daß die Wirkstoffe 1, 2, 7, 9, 10, 14, 21, 22, 28, 34 und 35 bei der Anwendung als 0,05%ige Spritzbrühe eine bessere fungizide Wirkung zeigen (90%) als das Vergleichsmittel (60%).

## Patentansprüche

1. 4-Aminopyrimidine der Formel

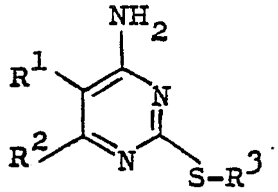

I,

worin

$R^1$ $C_5$—$C_{12}$-Alkyl oder im Phenyl gegebenenfalls durch $C_1$—$C_4$-Alkyl oder Halogen substituiertes Phenyl-$C_2$—$C_6$-alkyl,

$R^2$ Wasserstoff oder $C_1$—$C_4$-Alkyl,

$R^3$ $C_1$—$C_{12}$-Alkyl, $C_3$—$C_8$-Alkenyl, $C_3$—$C_8$-Alkinyl, $C_1$—$C_6$-Alkoxi-$C_1$—$C_4$-alkyl, $C_1$—$C_6$-Alkylthio-$C_1$—$C_4$- Cyanomethyl, $C_1$—$C_4$-Alkoxicarbonylmethyl, im Phenyl gegebenenfalls durch $C_1$—$C_4$-Alkyl, $C_1$—$C_4$- oder Halogen substituiertes Phenyl-$C_1$—$C_4$-alkyl, Furylmethyl, Tetrahydrofurylmethyl bedeutet.

erfahren zur Herstellung von 4-Aminopyrimidinen der Formel I, dadurch gekennzeichnet, daß man su ierte 3-Ketoester der Formel

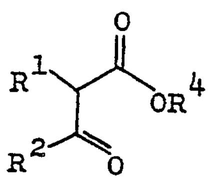

II

mit Thioharnstoff und Alkalialkoholat in Alkohol zu 2-Thiopyrimidonen der Formel

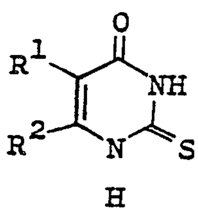

III,

kondensiert, wobei $R^1$ und $R^2$ die in Anspruch 1, angegebene Bedeutung haben und $R^4$ einen niederen Alkylrest bedeutet, diese mit einer Halogenverbindung $R^3$-Hal, in welcher $R^3$ die in Anspruch 1 angegebene Bedeutung hat und Hal Chlor, Brom oder Jod bedeutet, zu einem substituierten 2-Thiopyrimidon der Formel

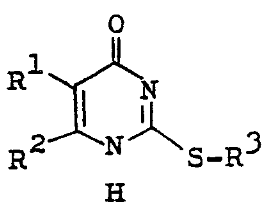

IV,

umsetzt, diese mit einem Chlorierungsmittel chloriert und das Chlorierungsprodukt mit Ammoniak unter Druck umsetzt.

3. Verfahren zur Bekämpfung von Pilzen, dadurch gekennzeichnet, daß man die Pilze oder die vor Pilzbefall zu schützenden Pflanzen, Saatgüter oder den Boden behandelt mit einer fungizid wirksamem Menge eines 4-Aminopyrimidins der Formel

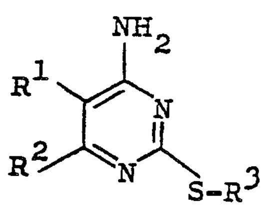

I,

worin

$R^1$ $C_5$—$C_{12}$-Alkyl oder im Phenyl gegebenenfalls durch $C_1$—$C_4$-Alkyl oder Halogen substituiertes Phenyl-$C_2$—$C_6$-alkyl,

$R^2$ Wasserstoff oder $C_1$—$C_4$-Alkyl,

$R^3$ $C_1$—$C_{12}$-Alkyl, $C_3$—$C_8$-Alkenyl, $C_3$—$C_8$-Alkinyl, $C_1$—$C_6$-Alkoxi-$C_1$—$C_4$-alkyl, $C_1$—$C_6$-Alkylthio-$C_1$—$C_4$-alkyl, Cyanomethyl, $C_1$—$C_4$-Alkoxicarbonylmethyl, im Phenyl gegebenenfalls durch $C_1$—$C_4$-Alkyl, $C_1$—$C_4$-Alkoxi oder Halogen substituiertes Phenyl-$C_1$—$C_4$-alkyl, Furylmethyl, Tetrahydrofurylmethyl bedeutet.

4. Fungizides Mittel, enthaltend einen üblichen Trägerstoff und ein 4-Aminopyrimidin der Formel

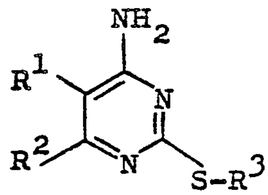

I,

worin

$R^1$ $C_5$—$C_{12}$-Alkyl oder im Phenyl gegebenenfalls durch $C_1$—$C_4$-Alkyl oder Halogen substituiertes Phenyl-$C_2$—$C_6$-alkyl,

$R^2$ Wasserstoff oder $C_1$—$C_4$-Alkyl,

$R^3$ $C_1$—$C_{12}$-Alkyl, $C_3$—$C_8$-Alkenyl, $C_3$—$C_8$-Alkinyl, $C_1$—$C_6$-Alkoxi-$C_1$—$C_4$-alkyl, $C_1$—$C_6$-Alkylthio-$C_1$—$C_4$-alkyl, Cyanomethyl, $C_1$—$C_4$-Alkoxicarbonylmethyl, im Phenyl gegebenenfalls durch $C_1$—$C_4$-Alkyl, $C_1$—$C_4$-Alkoxi oder Halogen substituiertes Phenyl-$C_1$—$C_4$-alkyl, Furylmethyl, Tetrahydrofurylmethyl bedeutet.

## Revendications

1. 4-aminopyrimidines de formule

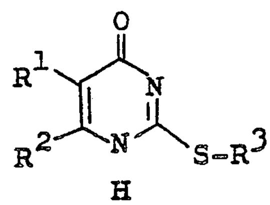

I,

dans laquelle

$R^1$ représente alkyle en $C_5$—$C_{12}$ ou phényl-alkyle en $C_2$—$C_6$ éventuellement substitué dans phényle par alkyle en $C_1$—$C_4$ ou halogène,

$R^2$ hydrogène ou alkyle en $C_1$—$C_4$,

$R^3$ alkyle en $C_1$—$C_{12}$, alcényle en $C_3$—$C_8$, alkinyle en $C_3$—$C_8$, alcoxy en $C_1$—$C_6$-alkyle en $C_1$—$C_4$, alkylthio en $C_1$—$C_6$-alkyle en $C_1$—$C_4$, cyanométhyle, alcoxy en $C_1$—$C_4$-carbonylméthyle, phényl-alkyle en $C_1$—$C_4$ éventuellement substitué dans phényle par alkyle en $C_1$—$C_4$, alcoxy en $C_1$—$C_4$ ou halogène, furylméthyle, tétrahydrofurylméthyle.

2. Procédé de préparation de 4-aminopyrimidines de formule I, caractérisé par le fait que l'on condense des 3-cétoesters substitués de formule

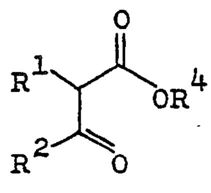

II

avec de la thiourée et de l'alcoolate alcalin dans de l'alcool, pour obtenir des 2-thiopirimidones de formule

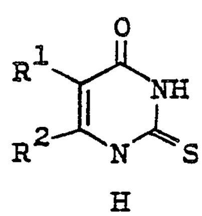

III,

$R^1$ et $R^2$ ayant les significations données dans la revendication 1 et $R^4$ représente un reste alkyle inférieur,

on fait réagir cette 2-thiopyrimidone avec un composé halogéné $R^3$-Hal, où $R^3$ a la signification donnée dans la revendication 1 et Hal représente chlore, brome, ou iode, pour obtenir une 2-thiopyrimidone substituée de formule

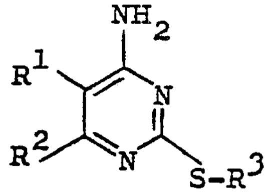

IV,

on chlore celle-ci avec un agent de chloration et on fait réagir sous pression le produit de chloration avec de l'ammoniac.

3. Procédé de lutte contre les champignons, caractérisé par le fait que l'on traite les champignons ou les plantes, semences ou le sol à protéger contre l'attaque de champignons avec une quantité efficace du point de vue fongicide d'une 4-aminopyrimidine de formule

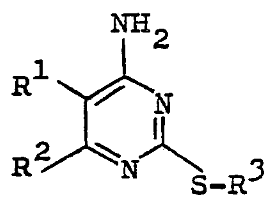

I,

dans laquelle

$R^1$ représente alkyle en $C_5$—$C_{12}$ ou phényl-alkyle en $C_2$—$C_6$ éventuellement substitué dans phényle par alkyle en $C_1$—$C_4$ ou halogène,

$R^2$ hydrogène ou alkyle en $C_1$—$C_4$,

$R^3$ alkyle en $C_1$—$C_{12}$, alcényle en $C_3$—$C_8$, alkinyle en $C_3$—$C_8$, alcoxy en $C_1$—$C_6$-alkyle en $C_1$—$C_4$, alkylthio en $C_1$—$C_6$-alkyle en $C_1$—$C_4$, cyanométhyle, alcoxy en $C_1$—$C_4$-carbonylméthyle, phényl-alkyle en $C_1$—$C_4$ éventuellement substitué dans phényle par alkyle en $C_1$—$C_4$, alcoxy en $C_1$—$C_4$ ou halogène, furylméthyle, tétrahydrofurylméthyle.

4. Agent fongicide, contenant un support usuel et une 4-aminopyrimidine de formule

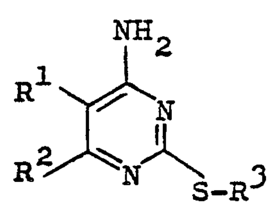

I,

dans laquelle

$R^1$ représente alkyle en $C_6$—$C_{12}$ ou phényl-alkyle en $C_2$—$C_6$ éventuellement substitué dans phényle par alkyle en $C_1$—$C_4$ ou halogène,

$R^2$ hydrogène ou alkyle en $C_1$—$C_4$,

$R^3$ alkyle en $C_1$—$C_{12}$, alcényle en $C_3$—$C_8$, alkinyle en $C_3$—$C_8$, alcoxy en $C_1$—$C_6$-alkyle en $C_1$—$C_4$, alkylthio en $C_1$—$C_6$-alkyle en $C_1$—$C_4$, cyanométhyle, alcoxy en $C_1$—$C_4$-carbonylméthyle, phényl-alkyle en $C_1$—$C_4$ éventuellement substitué dans phényle par alkyle en $C_1$—$C_4$, alcoxy en $C_1$—$C_4$ ou halogène, furylméthyle, tétrahydrofurylméthyle.

**Claims**

1. A 4-aminopyrimidine of the formula

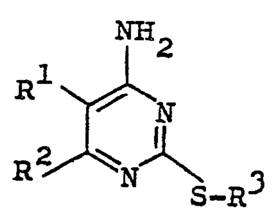

I,

where

$R^1$ is $C_5$—$C_{12}$-alkyl or is phenyl-$C_2$—$C_6$-alkyl which is unsubstituted or substituted in the phenyl moiety by $C_1$—$C_4$-alkyl or halogen,

$R^2$ is hydrogen or $C_1$—$C_4$-alkyl and
$R^3$ is $C_1$—$C_{12}$-alkyl, $C_3$—$C_8$-alkenyl, $C_3$—$C_8$-alkynyl, $C_1$—$C_6$-alkoxy-$C_1$—$C_4$-alkyl, $C_1$—$C_6$-alkylthio-$C_1$—$C_4$-alkyl, cyanomethyl or $C_1$—$C_4$-alkoxycarbonylmethyl or is phenyl-$C_1$—$C_4$-alkyl which is unsubstituted or substituted in the phenyl moiety by $C_1$—$C_4$-alkyl, $C_1$—$C_4$-alkoxy or halogen, or is furylmethyl or tetrahydrofurylmethyl.

2. A process for the preparation of a 4-aminopyrimidine of the formula I, wherein a substituted 3-ketoester of the formula

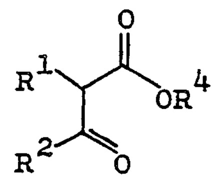

II

is condensed with thiourea and an alkali metal alcoholate in an alcohol to give a 2-thiopyrimidone of the formula

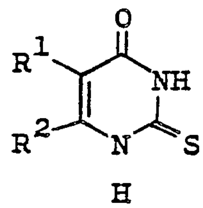

III,

where $R^1$ and $R^2$ have the meanings stated in claim 1 and $R^4$ is lower alkyl, the 2-thiopyrimidone is reacted with a halogen compound $R^3$-Hal, where $R^3$ has the meanings stated in claim 1 and Hal is chlorine, bromine or iodine, to give a substituted 2-thiopyrimidone of the formula

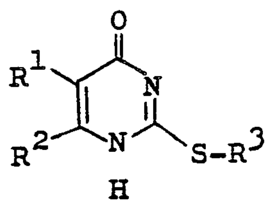

IV,

and the latter is chlorinated with a chlorinating agent and the chlorination product is reacted with ammonia under superatmospheric pressure.

3. A method for controlling fungi, wherein the fungi or the plants, seed or soil to be protected from fungal attack is or are treated with a fungicidal amount of a 4-aminopyrimidine of the formula

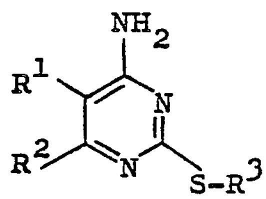

I,

where
$R^1$ is $C_5$—$C_{12}$-alkyl or is phenyl-$C_2$—$C_6$-alkyl which is unsubstituted or substituted in the phenyl moiety by $C_1$—$C_4$-alkyl or halogen,
$R^2$ is hydrogen or $C_1$—$C_4$-alkyl and
$R^3$ is $C_1$—$C_{12}$-alkyl, $C_3$—$C_8$-alkenyl, $C_3$—$C_8$-alkynyl, $C_1$—$C_6$-alkoxy-$C_1$—$C_4$-alkyl, $C_1$—$C_6$-alkylthio-$C_1$—$C_4$-alkyl, cyanomethyl or $C_1$—$C_4$-alkoxycarbonylmethyl or is phenyl-$C_1$—$C_4$-alkyl which is unsubstituted or substituted in the phenyl moiety by $C_1$—$C_4$-alkyl, $C_1$—$C_4$-alkoxy or halogen, or is furylmethyl or tetrahydrofurylmethyl.

4. A fungicide containing a conventional carrier and a 4-aminopyrimidine of the formula

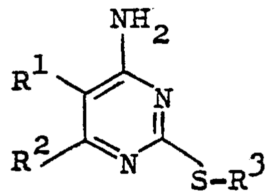

I,

where

$R^1$ is $C_5$—$C_{12}$-alkyl or is phenyl-$C_2$—$C_6$-alkyl which is unsubstituted or substituted in the phenyl moiety by $C_1$—$C_4$-alkyl or halogen,

$R^2$ is hydrogen or $C_1$—$C_4$-alkyl and

$R^3$ is $C_1$—$C_{12}$-alkyl, $C_3$—$C_8$-alkenyl, $C_3$—$C_8$-alkynyl, $C_1$—$C_6$-alkoxy-$C_1$—$C_4$-alkyl, $C_1$—$C_6$-alkylthio-$C_1$—$C_4$-alkyl, cyanomethyl or $C_1$—$C_4$-alkoxycarbonylmethyl or is phenyl-$C_1$—$C_4$-alkyl which is unsubstituted or substituted in the phenyl moiety by $C_1$—$C_4$-alkyl, $C_1$—$C_4$-alkoxy or halogen, or is furylmethyl or tetrahydrofurylmethyl.